# FASCICULE DE BREVET EUROPEEN

(11) **EP 1 030 614 B1**
(45) Date de publication et mention de la délivrance du brevet: **05.03.2003**
(21) Numéro de dépôt: 98955641.0
(22) Date de dépôt: 06.11.1998
(51) Int. Cl.: A61B 17/70

(54) **IMPLANT POUR VERTEBRE**
WIRBELSÄULENIMPLANTAT
VERTEBRA IMPLANT

(30) Priorité: 10.11.1997 FR 9714091
(43) Date de publication de la demande: 30.08.2000
(73) Titulaire: Dimso Distribution Medicale du Sud Ouest, 33610 Cestas (FR)
(72) Inventeur: SENEGAS, Jacques, F-33700 Mérignac (FR)
(74) Mandataire: Le Forestier, Eric
(86) Numéro de dépôt international: FR9802378
(87) Numéro de publication internationale: WO99023963

(56) Documents cités:
- WO-A-97/09940
- E.FRANK ET T.L.KEENEN: "A technique for cervical laminoplasty using mini plates" BRITISH JOURNAL OF NEUROSURGERY, vol. 8, no. 2, 1994, pages 197-199, XP002071037

## Description

L'invention concerne les cas de compression canalaire au niveau du rachis cervical.

Une compression canalaire au niveau des vertèbres cervicales peut survenir du fait d'un traumatisme ou d'une malformation.

Un but de l'invention est de fournir un dispositif permettant de répondre aux cas de compression canalaire.

Un tel dispositif est, par exemple, divulgué dans WO-A-97/09940.

En vue de la réalisation de ce but, on prévoit selon l'invention un implant pour vertèbre cervicale comportant deux branches adaptées à respectivement s'étendre sensiblement le long de deux lames cervicales d'une même vertèbre cervicale écartées l'une de l'autre après ostéotomie des lames, et un corps rigide reliant les deux branches.

Ainsi, après ostéotomie des lames cervicales et écartement de celles-ci vers l'arrière, on peut fixer les branches de l'implant aux lames respectives pour les maintenir écartées, ce qui met fin à la compression du canal.

Avantageusement, l'implant comporte une liaison déformable entre le corps et au moins l'une des branches.

Ainsi, l'implant s'adapte convenablement à la morphologie de la vertèbre.

Avantageusement, la liaison est flexible élastiquement.

Avantageusement, le corps comporte une zone ayant une épaisseur inférieure à une épaisseur du reste du corps et définissant la liaison.

Avantageusement, le corps a une forme générale courbe ayant un centre de courbure situé du côté des branches.

Ainsi, l'encombrement de l'implant est adapté à la forme du canal.

Avantageusement, le corps présente une face interne courbe située en regard des branches et une face externe opposée aux branches, la face interne présentant une courbure ayant un centre de courbure situé du côté des branches et un rayon de courbure inférieur à un rayon de courbure de la face externe.

Ici encore, la forme de l'implant ménage le volume nécessaire au canal.

Avantageusement, au moins l'une des branches présente des reliefs.

Avantageusement, les reliefs forment butée à l'encontre d'un déplacement le long de la branche, d'éléments de liaison s'étendant sensiblement perpendiculairement à une direction longitudinale de la branche.

Ainsi, on facilite la fixation des branches aux lames et on accroît la longévité de ces fixations.

Avantageusement, les reliefs comprennent des encoches.

Avantageusement, les branches sont aptes à être engagées dans des trous formés dans les lames cervicales, et les reliefs comprennent des dents formant harpons.

Avantageusement, la branche présente une largeur mesurée à partir d'un sommet des dents, décroissante en direction d'une extrémité libre de la branche.

Avantageusement, les reliefs s'étendent sur deux faces longitudinales de la branche opposées l'une à l'autre.

D'autres caractéristiques et avantages de l'invention apparaîtront encore dans la description suivante de deux modes préférés de réalisation donnés à titre d'exemples non limitatifs. Aux dessins annexés :
- la figure 1 et la figure 2 sont deux vues en élévation et de côté d'un implant selon un premier mode de réalisation de l'invention ;
- la figure 3 est une vue de l'implant de la figure 1 installé sur une vertèbre ;
- les figures 4 et 5 sont deux vues analogues aux figures 1 et 2 d'un implant selon un deuxième mode de réalisation de l'invention.

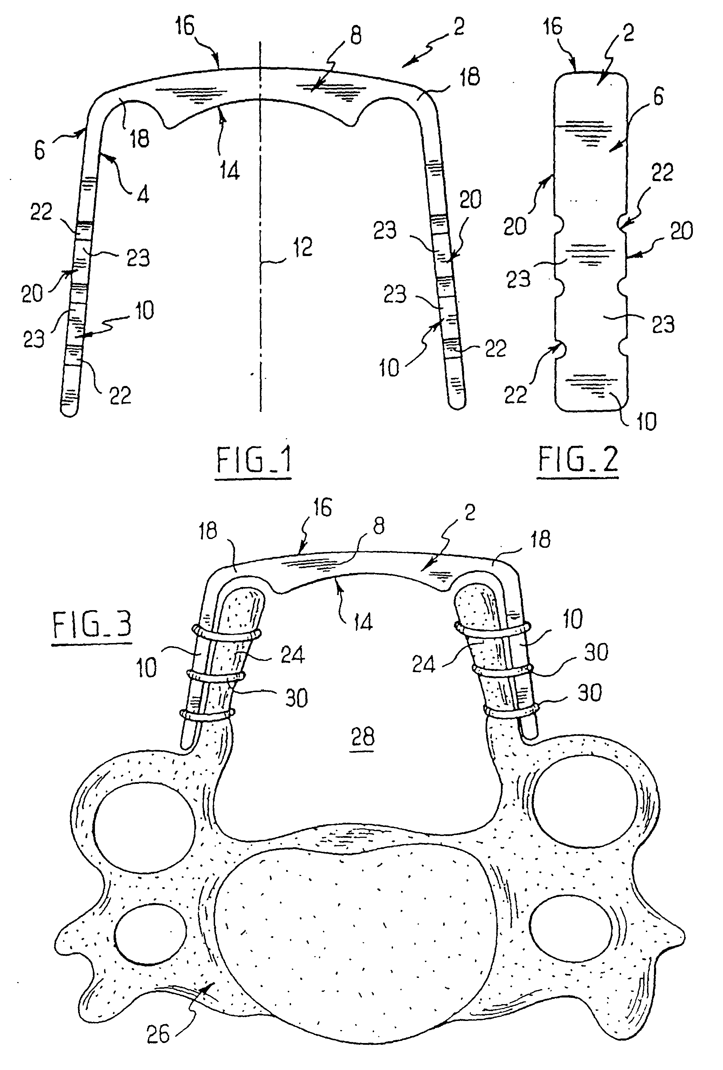

En références aux figures 1 et 2, dans un premier mode de réalisation, l'implant 2 a une forme générale de barre allongée plate présentant deux faces planes 4, 6 opposées l'une à l'autre et repliée pour lui donner une configuration en « U » en délimitant deux branches 8 et un corps 10 reliant les deux branches. La barre est repliée autour de deux axes, correspondant aux plis, et parallèles aux faces planes 4, 6 qui peuvent ainsi être appelées respectivement faces interne et externe. L'implant présente un axe de symétrie 12 passant par le milieu du corps 8. Les deux branches 10 sont inclinées vers l'extérieur chacune en direction opposée à cet axe 12. Le corps 8 et les deux branches 10 ont environ la même longueur.

Le corps 8 a une forme générale courbe ayant un centre de courbure situé sur l'axe 12 du côté des branches 10. Une face interne 14 du corps a un rayon de courbure inférieur à un rayon de courbure d'une face externe 16 du corps opposée à celle-ci. Le corps 8 présente deux zones de liaison 18 aux extrémités respectives du corps, contiguës aux branches 10. Les deux zones 18 sont amincies en présentant une épaisseur inférieure à une épaisseur du reste du corps. Elles constituent chacune une liaison souple déformable flexible élastiquement entre le corps 8 et chaque branche 10. Ces zones amincies 18 sont définies par une échancrure de la face interne 4 qui permet de ménager un espace pour la réception de l'extrémité des lames de la vertèbre.

Chaque branche 10 présente deux faces longitudinales de bord 20, dans lesquelles sont ménagées des paires d'encoches 22, les encoches de chaque paire s'étendant sur les faces 20 respectives à un même niveau le long de la branche. Les paires d'encoches 22 sont par exemple au nombre de trois sur chaque branche. Les encoches 22 définissent entre elles des dents 23 à sommet plat.

Pour utiliser l'implant, on pratique une ostéotomie des deux lames cervicales 24 d'une vertèbre cervicale 26 afin d'ouvrir le canal cervical 28 délimité par les lames. Puis on écarte les lames 24 l'une de l'autre vers l'arrière du corps du patient, par exemple jusqu'à leur donner seulement une légère inclinaison en direction l'une de l'autre, ce qui supprime la compression du canal 28.

On met en place l'implant 2. Pour cela, on applique chaque branche 10 le long d'une lame 24 respective, sur une face externe de la lame opposée à l'autre lame. On attache chaque branche 10 à la lame 24 associée par des moyens de cerclage 30 pouvant être des câbles ou des fils en matériau implantable. Ces moyens sont reçus dans les paires d'encoches respectives 22, ce qui interdit leur glissement le long de la branche 10 associée. Ainsi, on immobilise l'implant 2 sur son site d'implantation. L'implant permet de maintenir les lames 24 écartées l'une de l'autre.

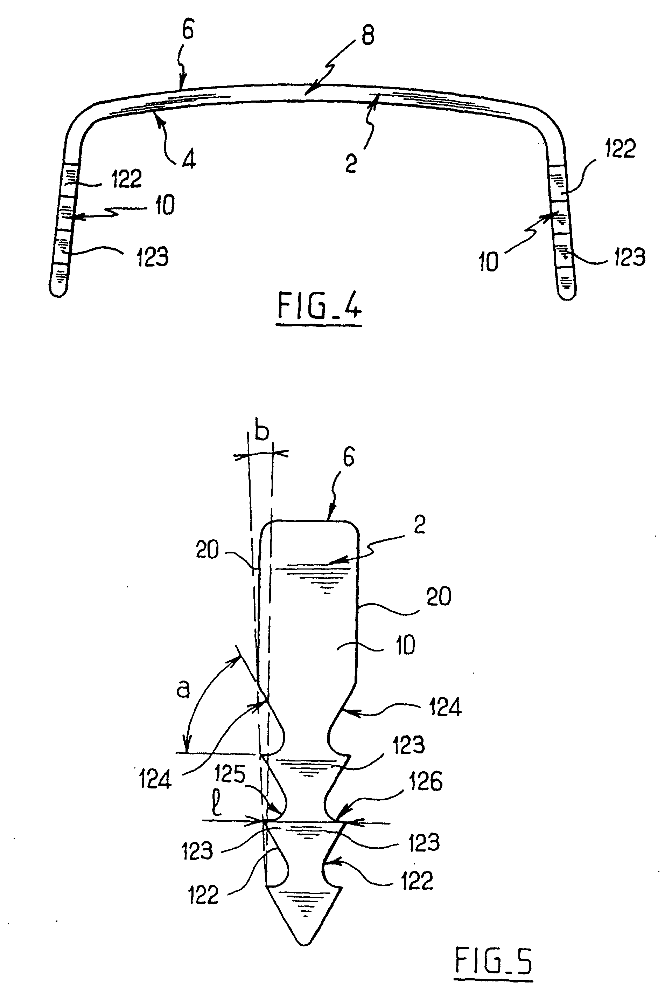

En référence aux figures 4 et 5, dans un deuxième mode de réalisation de l'implant, le corps 8 a une longueur environ égale à trois fois celles des branches 10. De plus, le corps 8 a une épaisseur constante sur toute sa longueur, identique à celle des branches 10. Il est délimité par les deux faces 4, 6 qui sont courbes, concentriques et de même rayon de courbure.

Chaque branche 10 présente des encoches 122 sur ses faces de bord 20, définissant des paires de dents 123 à sommet pointu. Chaque dent 123 présente du côté de l'extrémité libre de la branche 10 une face 124 inclinée d'un angle a par rapport à la direction perpendiculaire à la direction longitudinale de la branche. L'angle a vaut par exemple 60°. Chaque dent 123 présente du côté du corps 8 une face 126 perpendiculaire à la direction longitudinale de la branche. Au fond de l'encoche 122, la jonction 125 entre deux faces 124, 126 de dents 123 adjacentes est courbe. De plus, la largeur 1 de la branche 10 correspondant à la distance entre les sommets des dents 123 de chaque paire va en se rétrécissant linéairement depuis l'extrémité de la branche reliée au corps jusqu'à l'extrémité libre de la branche. Les sommets alignés des trois dents 123 de chaque face 20 définissent une ligne formant un angle b par rapport à la direction longitudinale de la branche 10. Cet angle b vaut par exemple 3°. La forme de réalisation des figures 4 et 5 s'applique avantageusement au cas où les deux branches 10 sont engagées dans des trous pratiqués en biais dans les lames cervicales, les dents 123 formant des harpons permettant d'éviter que lesdites branches se séparent des lames en sortant des trous précités,

## Revendications

1. Implant (2) pour vertèbre cervicale (26), **caractérisé en ce qu'**il comporte deux branches (10) adaptées à respectivement s'étendre sensiblement le long de deux lames cervicales (24) d'une même vertèbre cervicale (26) écartées l'une de l'autre après ostéotomie des lames, et un corps rigide (8) reliant les deux branches (10).

2. Implant selon la revendication 1, **caractérisé en ce qu'**il comporte une liaison déformable (18) entre le corps (8) et au moins l'une des branches (10).

3. Implant selon la revendication 2, **caractérisé en ce que** la liaison (18) est flexible élastiquement.

4. Implant selon la revendication 2 ou 3, **caractérisé en ce que** le corps (8) comporte une zone (18) ayant une épaisseur inférieure à une épaisseur du reste du corps et définissant la liaison.

5. Implant selon l'une quelconques des revendications 1 à 4, **caractérisé en ce que** le corps (8) a une forme générale courbe ayant un centre de courbure situé du côté des branches (10).

6. Implant selon l'une quelconque des revendications 1 à 5, **caractérisé en ce que** le corps (8) présente une face interne courbe (14) située en regard des branches (10) et une face externe (16) opposée aux branches, la face interne (14) présentant une courbure ayant un centre de courbure situé du côté des branches (10) et un rayon de courbure inférieur à un rayon de courbure de la face externe (16).

7. Implant selon l'une quelconque des revendications 1 à 6, **caractérisé en ce qu'**au moins l'une des branches (10) présente des reliefs (22, 23 ; 122, 123).

8. Implant selon la revendication 7, **caractérisé en ce que** les reliefs (22, 23 ; 122, 123) forment butée à l'encontre d'un déplacement le long de la branche (10), d'éléments de liaison (30) s'étendant sensiblement perpendiculairement à une direction longitudinale de la branche.

9. Implant selon la revendication 8, **caractérisé en ce que** les reliefs comprennent des encoches (22 ; 122).

10. Implant selon la revendication 7, **caractérisé en ce que** les branches (10) sont aptes à être engagées dans des trous pratiqués dans les lames cervicales, et **en ce que** les reliefs comprennent des dents (123) formant harpons.

11. Implant selon la revendication 10, **caractérisé en ce que** la branche (10) présente une largeur (1) mesurée à partir d'un sommet des dents (123), décroissante en direction d'une extrémité libre de la branche.

12. Implant selon l'une quelconque des revendications 7 à 11, **caractérisé en ce que** les reliefs s'étendent sur deux faces longitudinales (20) de la branche (10) opposées l'une à l'autre.

## Claims

1. An implant (2) for a cervical vertebra (26), the implant being **characterized in that** it comprises two branches (10) adapted to extend respectively substantially along the two cervical laminae (24) of a given cervical vertebra (26) that have been spaced apart from each other after osteotomy of the laminae, and a rigid web (8) interconnecting the two branches (10).

2. An implant according to claim 1, **characterized in that** it has a deformable link (18) between the web (8) and at least one of the branches (10).

3. An implant according to claim 2, **characterized in that** the link (18) is elastically flexible.

4. An implant according to claim 2 or 3, **characterized in that** the web (8) has a zone (18) of thickness that is reduced compared with the thickness of the remainder of the web and that defines the link.

5. An implant according to any one of claims 1 to 4, **characterized in that** the web (8) is generally curved in shape, having a center of curvature situated on the same side as the branches (10).

6. An implant according to any one of claims 1 to 5, **characterized in that** the web (8) has a curved inner face (14) situated facing the branches (10) and an outer face (16) opposite from the branches, the inner face (14) having curvature with a center of curvature that is situated on the same side as the branches (10) and a radius of curvature that is smaller than the radius of curvature of the outer face (16).

7. An implant according to any one of claims 1 to 6, **characterized in that** at least one of the branches (10) has portions in relief (22, 23; 122, 123).

8. An implant according to claim 7, **characterized in that** the portions in relief (22, 23; 122, 123) form abutments preventing link elements (30) that extend substantially perpendicularly to the longitudinal direction of the branch (10) from moving along the branch.

9. An implant according to claim 8, **characterized in that** the portions in relief comprise notches (22; 122).

10. An implant according to claim 7, **characterized in that** the branches (10) are suitable for being engaged in holes formed in the cervical laminae, and **in that** the portions in relief comprise barb-forming teeth (123).

11. An implant according to claim 10, **characterized in that** the branch (10) is of a width (*l*) measured between the tips of the teeth (123) that tapers towards the free end of the branch.

12. An implant according to any one of claims 7 to 11, **characterized in that** the portions in relief extend over two opposite longitudinal faces (20) of the branch (10).

## Patentansprüche

1. Implantat (2) für Halswirbel (26), **dadurch gekennzeichnet, daß** es zwei Arme (10), die dafür eingerichtet sind, sich jeweils praktisch entlang von zwei Zervikalblättern (24) eines selben Halswirbels (26) zu erstrecken, welche nach einer Osteotomie der Blätter voneinander beabstandet angeordnet sind, und einen festen Körper (8) aufweist, der die beiden Arme (10) verbindet.

2. Implantat gemäß Anspruch 1, **dadurch gekennzeichnet, daß** es eine verformbare Verbindung (18) zwischen dem Körper (8) und wenigstens einem der Arme (10) aufweist.

3. Implantat gemäß Anspruch 2, **dadurch gekennzeichnet, daß** die Verbindung (18) elastisch biegsam ist.

4. Implantat gemäß Anspruch 2 oder 3, **dadurch gekennzeichnet, daß** der Körper (8) einen Bereich (18) aufweist, der eine geringere Dicke aufweist als eine Dicke des restlichen Körpers, und der die Verbindung definiert.

5. Implantat gemäß irgendeinem der Ansprüche 1 bis 4, **dadurch gekennzeichnet, daß** der Körper (8) eine allgemein gekrümmte Form mit einem auf der Seite der Arme (10) angeordneten Krümmungsmittelpunkt aufweist.

6. Implantat gemäß irgendeinem der Ansprüche 1 bis 5, **dadurch gekennzeichnet, daß** der Körper (8) eine gegenüber der Arme (10) angeordnete gekrümmte Innenfläche (14) und eine den Armen entgegengesetzt angeordnete Außenfläche (16) aufweist, wobei die Innenfläche (14) eine Krümmung aufweist, die einen auf der Seite der Arme (10) angeordneten Krümmungsmittelpunkt und einen kleineren Krümmungsradius als ein Krümmungsradius der Außenfläche (16) aufweist.

7. Implantat gemäß irgendeinem der Ansprüche 1 bis 6, **dadurch gekennzeichnet, daß** wenigstens einer der Arme (10) Erhöhungen (22, 23; 122, 123) aufweist.

8. Implantat gemäß Anspruch 7, **dadurch gekennzeichnet, daß** die Erhöhungen (22, 23; 122, 123) einen Anschlag entgegen einer Bewegung entlang des Armes (10) für Verbiundungselemente (30), die sich praktisch senkrecht zu einer Längsrichtung des Armes erstrecken, bilden.

9. Implantat gemäß Anspruch 8, **dadurch gekennzeichnet, daß** die Erhöhungen Einkerbungen (22; 122) aufweisen.

10. Implantat gemäß Anspruch 7, **dadurch gekennzeichnet, daß** die Arme (10) dafür geeignet sind, in Löcher einzugreifen, welche in den Zervikalblättern ausgeführt sind, und daß die Erhöhungen ankerförmige Zähne (123) aufweisen.

11. Implantat gemäß Anspruch 10, **dadurch gekennzeichnet, daß** der Arm (10) eine Breite (1) aufweist, die, gemessen von einer Spitze der Zähne (123), in Richtung eines freien Endes des Armes abnimmt.

12. Implantat gemäß irgendeinem der Ansprüche 7 bis 11, **dadurch gekennzeichnet, daß** sich die Erhöhungen auf zwei einander gegenüberliegenden Längsflächen (20) des Armes (10) erstrecken.
